# NEW EUROPEAN PATENT SPECIFICATION

(11) **EP 1 906 883 B2**
(45) Date of publication and mention of the opposition decision: **02.12.2026**
(45) Mention of the grant of the patent: 27.01.2021
(21) Application number: 06771018.6
(22) Date of filing: 24.05.2006
(51) Int. Cl.: A61F 2/24

(54) **A NON-CYLINDRICAL PROSTHETIC VALVE SYSTEM FOR TRANSLUMINAL DELIVERY**
NICHTZYLINDRISCHES PROTHESENKLAPPENSYSTEM ZUR TRANSLUMINALEN ABGABE
SYSTEME DE VALVE PROTHETIQUE NON CYLINDRIQUE DESTINE A ETRE INTRODUIT PAR VOIE TRANSLUMINALE

(30) Priority: 24.05.2005 US 684192 P; 15.05.2006 US 434506
(43) Date of publication of application: 09.04.2008
(60) Divisional application: 21152787.4 / 3 878 409
(73) Proprietor: Medtronic Ireland Manufacturing Unlimited Company, Dublin 2, D02 T380 (IE)
(72) Inventor: SEGUIN, Jacques, Old Windsor Berkshire SL4 2NF (GB); BORTLEIN, Georg, 92190 Meudon (FR); NGUYEN, Than, Placentia, California 92870 (US); PANNEK, Edward, El Cajon, California 92019 (US)
(74) Representative: Zimmermann & Partner Patentanwälte mbB
(86) International application number: PCT/US2006/020012
(87) International publication number: WO 2006/127765

(56) References cited:
- WO-A1-02/36048
- WO-A1-2004/096100
- WO-A2-03/011195
- WO-A2-2006/124649
- US-A1- 2003 023 303
- US-A1- 2004 210 304
- ANONYMOUS: "Percutaneous Aortic Valve Replacement with a Self-Expanding Stent: The CoreValve ReValving™ Procedure", CATH LAB DIGEST, 1 November 2004 (2004-11-01), pages 26, 28 , XP055861513

## Description

### Field of the Invention

The present invention relates to a prosthetic cardiac valve and related deployment system that can be delivered percutaneously through the vasculature.

### Background of the Invention

Currently, the replacement of a deficient cardiac valve is often performed by opening the thorax, placing the patient under extracorporeal circulation or peripheral aorto-venous heart assistance, temporarily stopping the heart, surgically opening the heart, excising the deficient valve, and then implanting a prosthetic valve in its place. U.S. Patent No. 4,106,129 to Carpentier describes a bioprosthetic heart valve with compliant orifice ring for surgical implantation. This procedure generally requires prolonged patient hospitalization, as well as extensive and often painful recovery. It also presents advanced complexities and significant costs.

To address the risks associated with open heart implantation, devices and methods for replacing a cardiac valve by a less invasive means have been contemplated. For example, French Patent Application No. 99 14462 illustrates a technique and a device for the ablation of a deficient heart valve by percutaneous route, with a peripheral valvular approach. International Application (PCT) Nos. WO 93/01768 and WO 97/28807, as well as U.S. Patent Nos. 5,814,097 to Sterman et al., 5,370,685 to Stevens, and 5,545,214 to Stevens illustrate techniques that are not very invasive as well as instruments for implementation of these techniques.

U.S. Patent No. 3,671,979 to Moulopoulos and U.S. Patent No. 4,056,854 to Boretos describe a catheter-mounted artificial heart valve for implantation in close proximity to a defective heart valve. Both of these prostheses are temporary in nature and require continued connection to the catheter for subsequent repositioning or removal of the valve prosthesis, or for subsequent valve activation.

With regard to the positioning of a replacement heart valve, attaching this valve on a support with a structure in the form of a wire or network of wires, currently called a stent, has been proposed. This stent support can be contracted radially in such a way that it can be introduced into the body of the patient percutaneously by means of a catheter, and it can be deployed so as to be radially expanded once it is positioned at the desired target site. U.S. Patent No. 3,657,744 to Ersek discloses a cylindrical, stent-supported, tri-leaflet, tissue, heart valve that can be delivered through a portion of the vasculature using an elongate tool. The stent is mounted onto the expansion tool prior to delivery to the target location where the stent and valve are expanded into place. More recently, U.S. Patent No. 5,411,552 to Andersen also illustrates a technique of this type. In the Andersen patent, a stent-supported tissue valve is deliverable percutaneously to the native heart valve site for deployment using a balloon or other expanding device. Efforts have been made to develop a stent-supported valve that is self-expandable, using memory materials such as Nitinol.

The stent-supported systems designed for the positioning of a heart valve introduce uncertainties of varying degree with regard to minimizing migration from the target valve site. A cardiac valve that is not adequately anchored in place to resist the forces of the constantly changing vessel wall diameter, and turbulent blood flow therethrough, may dislodge itself, or otherwise become ineffective. In particular, the known stents do not appear to be suited to sites in which the cardiac wall widens on either proximally and/or distally of the valve annulus situs. Furthermore, the native cardiac ring remaining after ablation of the native valve can hinder the positioning of these stents. These known systems also in certain cases create problems related to the sealing quality of the replacement valve. In effect, the existing cardiac ring can have a surface that is to varying degrees irregular and calcified, which not only lessens the quality of the support of the stent against this ring but also acts as the source of leaks between the valve and this ring. Also, these systems can no longer be moved at all after deployment of the support, even if their position is not optimal. Furthermore, inflating a balloon on a stented valve as described by Andersen may traumatize the valve, especially if the valve is made from a fragile material as a living or former living tissue.

Also, the existing techniques are however considered not completely satisfactory and capable of being improved. In particular, some of these techniques have the problem of involving in any case putting the patient under extracorporeal circulation or peripheral aorto-venous heart assistance and temporary stopping of the heart; they are difficult to put into practice; they do not allow precise control of the diameter according to which the natural valve is cut, in view of the later calibration of the prosthetic valve; they lead to risks of diffusion of natural valve fragments, often calcified, into the organism, which can lead to an embolism, as well as to risks of perforation of the aortic or cardiac wall; they moreover induce risks of acute reflux of blood during ablation of the natural valve and risk of obstruction of blood flow during implantation of the device with a balloon expandable stent for example.

### Summary of the Invention

An embodiment of the present invention comprises a prosthesis frame comprising a plurality of structural members arranged to form cells of generally repeating cell patterns throughout the frame. In the preferred embodiment, the structural members are curved to distribute the mechanical stresses associated with frame expansion throughout the axial length of the structural members, rather than concentrating the stress at the junctions between the structural members, as with traditional stent designs having straight structural members. By distributing the mechanical stress of expansion, larger expansion ratios may be achieved, while reducing the risk of mechanical failure associated with larger expansion ratios. The structural members and cell configurations of the prosthesis frame may vary in one of more characteristics within the frame. In a preferred embodiment, larger cell sizes are provided in sections of the frame having larger expansion diameters, while smaller cell sizes are provided in sections of the frame having smaller expansion diameters. The heterogeneity of the cells may be manifested by differing cell sizes, cell shapes, and cell wall configurations and cross-sections.

In an embodiment of the invention, the prosthetic valve comprises a non-cylindrical prosthesis frame. Non-cylindrical frame shapes may be used to improve the anchoring and/or orientation of the prosthetic valve at the desired implantation site. In addition, a prosthesis frame may have one or more sections configured to expand to a restricted or preset diameter rather than to expand until restrained by surrounding anatomical structures. Control of the expansion diameter provides a portion of the prosthesis frame with a reproducible configuration irrespective of the surrounding anatomy. The reproducibility of valve geometry is enhanced in frames with controlled expansion diameters.

To further maintain the control of the expansion diameter of one or more portions of the prosthesis frame, mechanical effects from the variable expansion of adjacent portions of the prosthesis frame may be reduced by providing a stent with a curved outer surface that can distribute the mechanical force exerted by adjacent frame portions throughout the curved configuration and reduce any localized deformation may that result with a traditional cylindrical frame shape.

The implantation of the prosthetic valve may be performed with existing catheter and retaining sheath designs, as known in the art. To further facilitate implantation of such a device, additional delivery catheter features are also contemplated. These additional features include dual sheath withdrawal controls providing at least a slow and a fast sheath withdrawal, and an integrated introducing sheath. It is also contemplated that one or more longitudinal stiffening elements may be provided in the catheter or sheath walls to enhance the column strength and control of the delivery system, while preserving the bendability of the delivery system. To guide the tip of the catheter to a desired position, a proximally controllable steering wire may be provided on the catheter, or alternately, a separate snare may be used to engage and move the tip of the catheter or guidewire toward the desired position.

In one particular embodiment of the invention comprising a self-expandable prosthesis frame, it is contemplated that the device may be implanted into patients having existing prosthetic valves that were surgically or transluminally placed. Such a procedure cannot be performed with balloon-expandable prosthetic valves because the rigidity of the existing prosthetic valve prevents adequate overexpansion of the prosthetic valve to achieve anchoring of the balloon-expandable valve. Without overexpansion, once the balloon is released, the prosthesis frame tends to rebound and radially contract, thus requiring that balloon-expandable prostheses be overexpanded in order to achieve the desired final expansion configuration.

Although some embodiments of the invention are described using an example of a prosthetic valve for treatment of aortic valve disorders, prostheses configured for use in other cardiac valve or circulatory system positions or are also contemplated, including but not limited to those at the mitral, pulmonic and tricuspid valve positions. Valve implantation in any of a variety of congential cardiac malformations or other circulatory system disorders are also contemplated and may include implantation of valves into the aortic root, ascending aorta, aortic arch or descending aorta. It is also understood that the general prosthesis frame and valve may be incorporated into other types of medical devices, such as vascular grafts for abdominal aortic aneurysms.

In one embodiment, a prosthetic valve assembly is provided, comprising a prosthesis frame having a first and second end and having a reduced and expanded configuration, the frame comprising a first zone proximal the first end, a second zone proximal the second end, and a third zone therebetween, said zones positioned axially with respect to each other, wherein the fully expanded diameter of the first zone is different than that of the second zone; and a valve engaged to the prosthesis frame. The valve may be primarily supported by the third zone. The fully expanded diameter of the third zone may be less than those of the first and second zones. The third zone may comprise a generally concave portion. The prosthesis frame may be self-expanding. The first zone of the prosthesis frame may be tapered. The second zone may comprise a generally bulbous configuration. The first zone may comprise a generally tapered configuration. The first zone may be adapted to wedge against a patient's native valve leaflets and/or a patient's surgically implanted valve leaflets. The first zone may also be adapted to deflect one or more commissure posts of a surgically implanted heart valve. In some embodiments, no substantial continuous portion of the prosthesis frame is of constant diameter. The second end may have a diameter less than the greatest fully expanded diameter of the second zone. The prosthesis frame may comprise a plurality of cells defined by one or more structural members, wherein the cells that are configured so as to be expandable. A portion of the plurality of cells may be homogeneous in shape, heterogeneous in shape, homogeneous in size, heterogeneous in size, homogeneous in structural member configuration, and/or heterogeneous in structural member configuration. At least some of the structural members may have varied cross-sectional configurations along their length.

In another embodiment, a prosthetic valve assembly for treating a patient is provided, comprising a valve for controlling blood flow; a non-cylindrical means for maintaining and supporting the geometry of the valve means; and an anchor attached to the non-cylindrical means. The maintaining and supporting means may comprise a prosthesis frame comprising a plurality of expandable cells and having a non-uniform diameter along its length.

In another embodiment, a prosthetic valve assembly is provided, comprising a prosthesis frame having a first zone, a second generally bulbous zone having a maximum expanded diameter greater than that of the first zone, and a valve support zone having a maximum expanded diameter smaller than those of the first and second zones. The first zone may be tapered. The valve support zone may be generally concave in outer configuration. The valve assembly may further comprise a valve supported by the valve support zone. The valve may be a tri-cuspid tissue valve. The frame may be self-expandable. A method of implanting the valve assembly described above is also provided, the method comprising the steps of mounting the valve assembly onto a catheter suitable for percutaneous and vascular delivery and deploying said valve assembly within an appropriate native lumen of the patient. The step of deploying may comprise deploying the valve assembly within a previously-implanted prosthetic cardiac valve.

In an example, a method of implanting the valve assembly in a patient is provided, the method comprising providing a prosthetic valve assembly comprising a prosthesis frame having a first zone, a second generally bulbous zone having a maximum expanded diameter greater than that of the first zone, and a valve support zone having a maximum expanded diameter smaller than those of the first and second zones, said prosthetic valve assembly mounted onto a catheter suitable for percutaneous and vascular delivery and deploying said valve assembly within an appropriate native lumen of the patient. Deploying may comprise deploying the valve assembly within a previously-implanted prosthetic cardiac valve.

In one example, a method for treating a patient is provided, comprising inserting a self-expanding valve into the lumen of a previously-implanted cardiovascular device with a lumen of a patient. The implanted cardiovascular device may be a surgically implanted cardiac valve or an aorto-ventricular conduit. The method may further comprise expanding the self-expanding valve against one or more valve leaflets of a patient without contacting a valve annulus of the patient. The surgically implanted cardiac valve may comprise at least one commissure post and a bloodflow cross-sectional area. The method may further comprise outwardly deflecting the at least one commissure post. The method may further comprise deflecting the at least one commissure post to increase the bloodflow cross-sectional area. At least a portion of the at least one commissure post may be moved at least about 1 mm, at least about 1.5 mm, or at least about 2 mm. The previously-implanted cardiovascular device may comprise a valve leaflet support with a cross-sectional area. The method may further comprise deforming the valve leaflet support to increase the cross-sectional area. In some embodiments, at least a portion of the at least one commissure post is deflected at least about 3 degrees, at least about 5 degrees, or at least about 10 degrees. The at least a portion of the at least one commissure post may deflected from a generally radially inward position to a generally parallel position, or from a generally radially inward position to generally radially outward position.

In one example, a method for implanting a cardiovascular device is provided, comprising inserting an expandable heart valve into a vascular system of a patient, anchoring the expandable heart valve against a distal surface of one or more valve leaflets of the patient without contacting an annulus surface of the patient. The one or more valve leaflets may be native valve leaflets and/or artificial valve leaflets.

In one example, a method for treating a patient is provided, comprising inserting a self-expanding valve into the lumen of a previously-implanted cardiovascular device with the native lumen of a patient. The implanted cardiovascular device may be a surgically implanted cardiac valve or an aorto-ventricular conduit.

In another example, a method for implanting a cardiovascular device is provided, comprising providing a cardiovascular device located on a delivery system; inserting the delivery system through an aortic arch of a patient from a first arterial access point; inserting a snare from a second arterial access point; grasping the delivery system with the snare; and manipulating the snare to align the delivery system with a lumen of the patient's aortic valve. The cardiovascular device may be a self-expanding valve. The delivery system may comprise a catheter and guidewire, and/or a catheter and retaining sheath. The grasping step may comprise grasping the catheter with the snare or grasping the guidewire with the snare. The catheter may comprise a retaining sheath controller. The retaining sheath controller may comprise one or more detents or stops for a defined sheath position. The catheter may comprise a multi-rate retaining sheath controller, one or more longitudinal stiffening elements, a catheter circumference and two longitudinal stiffening elements located generally on opposite sides of the catheter circumference. The retaining sheath may comprise one or more longitudinal stiffening elements, and/or a retaining sheath circumference and two longitudinal stiffening elements located generally on opposite sides of the retaining sheath circumference. The delivery system may comprise a catheter and introducer sheath. The catheter may comprise a distal delivery section and a proximal body having a reduced diameter relative to the distal delivery section. The introducer sheath may be integrated with the proximal body of the catheter.

The above embodiments and methods of use are explained in more detail below.

### Brief Description of the Drawings

Figure 1 is a schematic view of one embodiment of a non-cylindrical prosthesis frame comprising elliptoid cells with variable sizes.
Figure 2 is a schematic view of the prosthesis frame of Figure 1 implanted in the aortic position.
Figure 3 depicts one embodiment of the invention comprising a delivery catheter inserted from an arterial access site and passed through the aortic arch.
Figure 4A depicts the use of a snare used to grasp the distal end of delivery catheter. Figure 4B illustrates the reorientation of the distal end of the delivery catheter toward the aortic valve lumen using the snare.
Figure 5A is a schematic view of a previously surgically implanted aortic valve in a patient. Figure 5B depicts the implantation of a self-expanding replacement aortic valve into the previously surgically implanted aortic valve.
Figure 6 is a schematic view of a patient with a previously surgically implanted aortic valve with deflected commissure posts and a replacement valve implanted within.
Figure 7 is a schematic view of an expandable prosthetic valve with a tapered inflow section.
Figure 8 is a schematic view of a patient with a self-expanding replacement aortic valve anchored about the leaflets of the existing valve leaflets.

### Detailed Description of the Preferred Embodiment

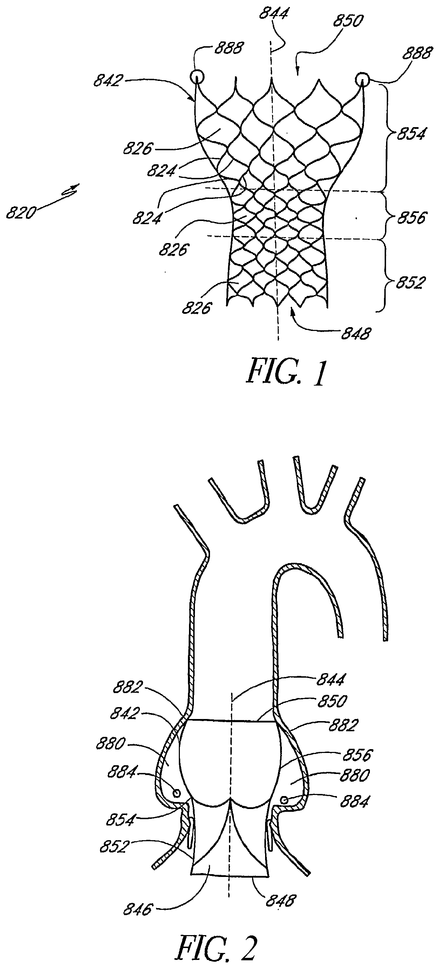

Referring to Figure 1, as well as Figure 2 showing application to (for example) an aortic valve and surrounding lumen, a particular prosthesis configuration is contemplated, exemplified by the embodiment shown therein, where such configuration has been shown to be very effective at supporting a prosthetic heart valve within a native lumen. With this contemplated configuration, as with other possible variations, a heterogeneous pattern of asymmetrical cells is provided, although portions thereof may comprise homogeneous patterns as well. With continuing reference to Figure 1 , one embodiment of the present invention comprises a heart valve prosthesis 820 comprising a non-cylindrical frame 822 having an intersecting pattern of structural members 824 that join to form cells 826 of varying sizes and shapes.

The non-cylindrical frame 822 of Figure 1 is shown in a fully expanded state with a longitudinal axis 844 therethrough. The heart valve prosthesis 820 further comprises, preferably and by way of example, a tricuspid tissue valve 846 supported by the frame 822. Improvements to a tricuspid tissue valve contemplated for use with the present invention are described in co-pending application Serial No. 11/128,826, entitled "HEART VALVE PROSTHESIS AND METHODS OF MANUFACTURE AND USE" and filed May 13, 2005. The non-cylindrical frame 822 comprises an inflow end 848 and an outflow end 850, with three zones therebetween: an inflow zone 852, an outflow zone 854 and a valve support zone 856 positioned between the inflow zone 852 and the outflow zone 854. The frame 822 is configured to be contracted to a much smaller size for, by way of example, insertion within a catheter sheath for deployment at the site of a heart valve.

The non-cylindrical frame 822 preferably comprises portions having homogeneous and heterogeneous patterns of cells. The homogeneous portion or portions may comprise a plurality of cells in which adjacent cells are of the same size, shape and/or wall (structural member) configuration. In one embodiment, exemplified by the one shown in Figure 1, each row of cells is homogeneous, although two or more adjacent rows could be homogeneous as well and still achieve the function of the particular embodiment shown. It is contemplated, however, that irregularity may be desired, in which case a row of heterogeneous cells may be beneficial. The homogeneous portion may also comprise a first alternating array of cells in which each first alternative cell is of the same shape, size and/or wall configuration, with a second alternating array of cells being different from the first but wherein each second alternative cell is of the same shape, size and/or wall configuration.

The heterogeneous portion or portions of the frame 822, at least in the embodiment exemplified in Figure 1 , may comprise a plurality of cells in which adjacent cells are not of the same size, shape and/or wall configuration. For example, even as between two cells having generally the same size, their relative length-to-width ratios may be different. Likewise, even as between two cells having generally the same shape, their relative sizes may be quite different. In one embodiment, exemplified by the one shown in Figure 1 , adjacent cells 826 along the longitudinal axis 844 (from the inflow end 848 to the outflow end 850) are different in size, shape and/or wall configuration. In this particular embodiment, the cells 826 are largest at the outflow zone 856, smaller at the inflow zone 852, and smallest at the valve support zone 854. Upon expansion, the shape of the various cells differs as well along the longitudinal axis. This variation in arrangement of cell size, shape and/or relative dimension permits dramatic differences in the degree of radial expansion of individual cells within the prosthesis frame. It is believed that relatively larger cell sizes generally allow greater radial expansion at such portions of the prosthesis frame while relatively smaller cell sizes generally limit or control the degree of radial expansion at those portions of the prosthesis frame. It is also believed that variations in the cross-section of individual structural members will also impact the degree of radial expansion and the radial force exerted against any lumen within which it is deployed. The heterogeneous portion may also consist of a plurality of alternating arrays or alternating rows of cells wherein a first set of alternating arrays or rows are homogeneous in shape, size and/or wall configuration but the balance are heterogeneous in shape, size and/or wall configuration.

With some embodiments, as exemplified by the one in Figures 1 and 2 , the inflow zone 852 may be tapered inwardly from inflow end 848 toward valve support zone 854. This generally conical configuration beneficially resists migration of the prosthesis frame against the forces generated by blood flow from the left ventricle to the aortic arch. The conical configuration is believed to provide increasing radial outward force and/or frictional resistance with surrounding structures when deployed in-situ. The configuration of the inflow end 848 may also be tailored to provide a mechanical abutting surface against the superior surface of the left ventricle 672 to resist displacement of the prosthesis. In the preferred embodiment, the increased radial outward force exerted by the inflow zone 868 may be provided through changes in the configuration of the cells and/or the structural members, or by particular cell arrangements. It would be expected that, based upon this teaching, one of ordinary skill in the art could optimize various parameters to create frames meeting particular needs.

With reference still to Figures 1 and 2 , in one embodiment of the invention, the valve support zone 854 is configured to support a valve, for example a tricuspid tissue valve 846. As explained above, it is both inventive and important for the portion of the supporting frame to have varied expansion and radial forces along the length of the frame. With this particular example, the valve support zone 854 is configured to ensure a controlled expansion upon deployment. Specifically, the cells 826 of the valve support zone 854 are arranged and/or configured to expand to a defined or preset maximum diameter. As explained above, controlling the expanded diameter of the portion of the frame supporting the valve provides greater control over coaptivity of the valve leaflets. That ensures that the valve 846 supported directly therein operates as effectively as possible in-situ. If the frame 822 at the valve support zone 854 were permitted to expand insufficiently, the leaflets might overlap to an undesirable degree, resulting in less efficient blood flow. A similar result would occur if the valve support zone were permitted to expand too much.

The valve support zone 854 comprises a generally axially-curved or concave configuration, or an overall toroidal configuration, as shown by example in Figure 1 . Such a configuration can further resist deviations from the desired or optimal valve support zone expansion configuration because variations in the mechanical stress exerted from the inflow zone 852 and/or outflow zone 856, caused by anatomical and pathological variations of surrounding structures, will be dispersed along the entire length of the middle zone curved structure, thereby minimizing or preventing any effects on middle zone expansion to its defined or optimal expansion configuration. In comparison, a prosthesis frame with a more cylindrical shape may respond more unpredictably to variations in a patient's anatomy by kinking or bowing, thereby disrupting the geometry of the valve that is resistant to expansion variations of adjacent zones. By providing a consistent expanded configuration for the valve support zone that is resistant to expansion changes of adjacent zones, a consistent valve geometry is achieved and valve function may be improved. Restricting one or more portions of the prosthesis frame to an expansion size that is generally less than the lumen of the surrounding anatomical structures and a range of potential anatomical variations may provide a prosthesis design with a reproducible valve configuration without unduly restricting the cross-sectional area of restriction frame expansion to the degree where the rate of blood flow is impaired.

As explained, the valve leaflets of valve 846 (or opening of any type of valve supported within the frame) are preferably positioned in the valve support zone 854 because the reproducibility and predictability of its cross sectional area and/or shape helps to maintain the desired valve geometry and coaptivity of those leaflets. In alternative embodiments of the invention, other portions of the valve assembly (e.g., commissure), may be located or engaged to the inflow zone 852 and/or outflow zone 856 to provide improved support and stability of the valve assembly along a greater portion of the prosthesis frame 822. A valve assembly spanning two or more zones of the prosthesis may help to disperse mechanical forces acting upon the valve assembly.

It is contemplated that with the present invention, for example as with the embodiment shown in Figure 2 , the valve support zone 854 of the frame 822 can be configured for supra-annular positioning above the aortic valve annulus when deployed; that is, the valve support zone 854, which supports prosthetic valve 846, is preferably positioned above the native valve. That provides at least two benefits: one, it permits a more controlled expansion of the valve support zone 854, unconstrained by the native lumen; and two, it provides more space for the valve opening or valve assembly as it is not constrained by the lumen of the native valve location which is often stenotic. Limited expansion of a prosthesis frame intended to occupy at least the supra-annular region may also be beneficial because it may prevent unnecessary expansion of the prosthesis frame 822 into other body structures. For example, by limiting expansion of the prosthesis frame 822 at the valve support zone 854 and providing a space 880 between the prosthesis frame 822 and the walls of the aortic root or bulb 882, occlusion of the coronary ostia 884 by the prosthesis frame 822 may be avoided. A sufficient space 880 between the frame 822 and the coronary ostia 884 would also permit access to the ostia 884 using coronary catheters to perform coronary catheterization for diagnostic or therapeutic purposes, if necessary, after deployment of the prosthesis frame 822. Coronary catheters can access the space 880 surrounding the prosthesis either through the cells in the cells 826 of the prosthesis frame 822 or other cells that may be provided in the prosthesis frame 822.

Referring still to Figures 1 and 2 by example, the valve support zone 854 and the outflow zone 856 of the prosthesis frame 822 may also be further configured with an increasing cross-sectional size along the longitudinal axis 844 in the direction away from the valve support zone 854 toward the outflow end 850. The purpose, among other reasons, for doing so is to resist migration or displacement caused by backflow forces of the column of blood in the ascending aorta. While it is commonly believed that aortic valve prosthesis migration is greater along the direction of forward blood flow, i.e. from the left ventricle to the aorta, there can be equal or greater forces applied by the backflow of blood following systole. The mass of blood flowing through the aortic valve during systole is generally equivalent to the stroke volume of the left ventricle, generally about 25 ml to about 75 ml, or greater if a patient has a dilated left ventricle 672 from aortic insufficiency. However, it is hypothesized that upon completion of the systolic phase of heart contraction, the backflow of blood that causes closure of the aortic valve is generated by the entire column of blood in the ascending aorta and aortic arch, which results in a much greater back flow force than the forward force exerted during systole. Thus, it is hypothesized that anchoring of the prosthesis frame may be optimized or improved using directional or non-directional anchoring or fixation structures that consider backflow forces as well as or more than forward migration forces. It should also be noted that the prosthesis frame embodiments disclosed herein may comprise discrete anchors positioned proximally, distally, or therebetween, to further enhance reduction, if not elimination, of migration in-situ.

It is contemplated that, as exemplified by the embodiments of Figures 1 and 2 , the present inventive prosthesis may comprise a non-uniform diameter frame, in which no substantial continuous portion of the prosthesis frame has a constant diameter. Moreover, the prosthesis frames described herein may be self-expandable or balloon expandable.

In one embodiment of the invention, the inflow end 848 of the prosthesis frame 822 in the expanded configuration has a diameter of about 15 mm to about 40 mm, preferably about 25 mm to about 30 mm, and most preferably about 26 mm or about 29 mm. In one embodiment, the outflow zone 856 of the prosthesis frame 822 in the expanded configuration has a maximum diameter of about 35 mm to about 65 mm, preferably about 40 mm to about 60 mm, and most preferably about 45 mm or about 55 mm. The restricted diameter of the valve support zone 854 of the prosthesis frame 822 may be about 18 mm to about 30 mm, preferably about 20 mm to about 28 mm, and most preferably about 22 mm or about 24 mm. Actual in situ or in vivo diameters in the expanded configurations may vary depending upon the anatomy and pathology of the individual patient.

It is contemplated that the prosthesis frame of any of these aforementioned embodiments may be manufactured using any of a variety of processes known in the art. Laser cutting of the prosthesis from metal tubular structure is one preferred method, but other methods such as fusing multiple wire elements together, or bending of one or more wire elements into a prosthesis frame may also be used. With laser cutting, the starting tube material may be of uniform diameter or of varied diameter, depending upon the desired fully expanded configuration desired. The slits or cells cut into the tube may be of uniform size or of varied size, again depending upon the desired expanded configuration.

As explained above, it is contemplated that the prosthesis frame 822 would be configured so that when deployed it could be positioned so as to be constrained at the native valve annulus by the anchoring function of the inflow zone 852, the upper portion of the prosthesis frame 822 could still be subject to unintended or undesired lateral movement due to the profile of the native lumen. To minimize such movement, the prosthesis frame 822 is preferably configured so that an enlarged radial cross-section at the outflow zone 856 would engage or be positioned so as to be close to engaging the adjacent wall of the native lumen. It is contemplated that if one makes the present invention as exemplified by the embodiment shown in Figures 1 and 2 , the outflow zone 856 of the prosthesis frame 822 would abut the aortic lumen along at least one or more portions of its perimeter to maintain the orientation of the prosthesis frame in a desired position.

An additional feature of at least the embodiments exemplified in Figures 1 and 85 is that the diameter of the prosthesis frame 822 at the outflow end 850 is smaller than the diameter within the outflow zone 856 adjacent thereto. In one specific embodiment, the outflow zone 856 comprises a generally bulbous structure intended to occupy a substantial portion of space in the aortic bulb 882 or ascending aorta. Having a generally bulbous configuration has a benefit of potentially minimizing trauma to the ascending aorta during deployment. As contemplated in deployment, the outflow end 850 could be the last portion of the prosthesis frame 822 released from a delivery catheter when the prosthesis is deployed through the aorta valve from a peripheral artery. Given the relatively large expansion ratio of the outflow zone 856 and the sudden rate of unconstrained self-expansion, it is contemplated that, in some situations, the outflow end 850 might pose a risk of damage to the lumen of the aorta. This risk may be reduced by tapering radially inwardly the outflow end 850 in the expanded configuration.

The present invention is suitable for placement at the aortic valve annulus, as shown in Figure 2. In that regard, the inflow zone 852 of the non-cylindrical frame 822 is configured, when implanted, to exert a radially outward force against surrounding structures in the expanded configuration of the frame. The radially outward force may push aside existing valve components, if needed, to enlarge the cross-sectional area available for blood flow through the valve. Although the native valve leaflets are shown in Figure 2 as having been pushed into the left ventricle, one or more leaflets may be pushed into the aorta. The radially outward force may also provide frictional resistance to prosthesis migration that may be caused by blood flow, cardiac muscle contraction and other factors.

Although the valve prosthesis may be implanted using a basic delivery catheter and retaining sheath, when a self-expanding structure is released from a retaining sheath and expanded, it has a tendency to pull out the remaining portions of the frame from between the catheter and sheath, resulting in a "springing out" or "jumping out" effect of self-expanded structures with premature deployment of the device. Referring to Figure 1 , the outflow zone 856 or outflow end 850 of the prosthesis frame 822 may further comprise one or more, and preferably two or more, engagement structures 888 for retaining a portion of the prosthesis frame 822 on the delivery catheter to allow partial release of the prosthesis frame in a controlled manner. The engagement structures 888 may also be useful for engaging a deployed prosthesis frame for the purposes of removing the device or repositioning the fully deployed device.

The delivery catheter and retaining sheath may comprise additional features to enhance the implantation of the prosthetic valve. In one embodiment, the retraction of the retaining sheath is actuated proximally on the catheter using a mechanical control, such as a dial or slide. The mechanical control may provide one or more detents or other type of stop mechanism at a point in sheath retraction where further retraction may result in a significant action such as the initial release of the prosthesis frame and/or release of the engagement structures, if any. The detents or stop may provide tactile feedback to the operator (i.e. temporary resistance to further movement) or require altered user intervention (i.e. shift direction or activate a button or latch) to further retract the sheath.

The delivery catheter and retaining sheath may comprise mechanical controls having different mechanical advantages for retracting the sheath. In one embodiment, a dial control may be provided on the proximal catheter to slowly withdraw the sheath, thereby allowing fine control of prosthesis release during the initial positioning of the device. Once the device is deployed to the extent where release of the remaining prosthesis would not substantially affect the desired valve location, a slide control may be used to quickly retract the rest of the sheath and to fully release the prosthesis.

As previously described, although the structural members of the prosthesis frame may be configured to provide greater expansion ratios compared to existing stent-type frames, due to the presence of the valve assembly in the prosthesis frame and the limited extent that the prosthetic valve profile in the delivery configuration may be reduced without damage to the valve assembly, the diameter of the delivery catheter loaded with the prosthetic valve may be larger compared to delivery catheters loaded with coronary stents. In some instances, the diameter of the delivery catheter may be sufficiently large to preclude the use of off-the-shelf introducer sheaths or to require a larger-than-desired opening into a blood vessel in order to use a sheath. It is recognized that only the distal portion of such a delivery catheter containing the prosthetic valve may have a larger diameter and that the sections or segments of the delivery catheter and retaining sheath proximal to the prosthetic valve may have a smaller diameter. However, once the enlarged diameter portion of the delivery catheter is initially inserted into an access site, an introducer sheath can no longer be inserted over the delivery catheter. To overcome this limitation, in some embodiments of the invention, an integrated introducer sheath may be provided with the delivery catheter that is capable of sliding along the delivery catheter body proximal to the portion containing the prosthetic valve. Once the prosthetic valve portion of the delivery catheter is inserted, the integrated introducer is then passed into access site along with the reduced diameter portion of the delivery catheter. Once the integrated introducer is fully inserted, the remaining portions of the delivery catheter can slide through the access site using the introducer. The integrated introducer may also have a peel-away feature that is known to those in the art such that it may be removed from the delivery catheter while the distal end of the delivery catheter remains in the body.

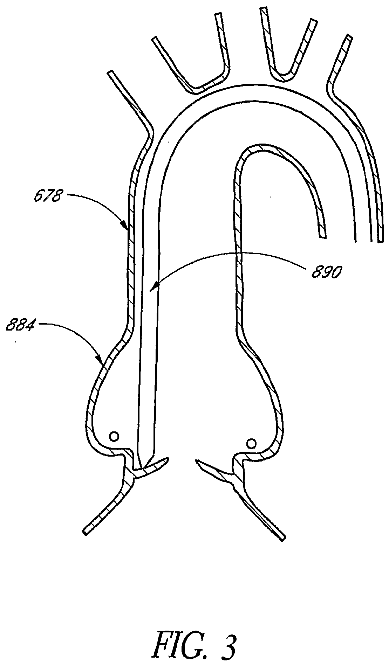

Because the distance from the insertion or access site on the body may be a substantial distance from the implantation site of the prosthetic valve, one or more longitudinal stiffening elements may be provided along the length of the delivery catheter and/or retaining sheath to provide sufficient "pushability" or column strength to adequately manipulate the distal end of the delivery catheter across the substantial distance. Such stiffening, however, may restrict the flexibility of the catheter. For example, when a prosthetic valve is inserted via a femoral artery and through the descending aorta to the aortic arch, the stiffness of the delivery catheter is likely to cause the delivery catheter to follow the path that generates the least amount of mechanical strain on the catheter body. With reference to Figure 3 , that results in a delivery catheter 890 that sits eccentrically in the lumen to one lateral side of the ascending aorta 678 or aortic bulb 884. Such a catheter may be difficult to manipulate and direct more centrally in the aortic lumen or through a stenotic aortic valve having a small central lumen. To provide a delivery catheter 890 with adequate column strength yet having sufficient flexibility to be manipulated with respect to the cross sectional lumen position, the longitudinal stiffening elements may be arranged about 180 degrees apart on the delivery catheter body or retaining sheath. This provides a plane of bending to the delivery catheter that lies between the two spaced apart stiffening elements.

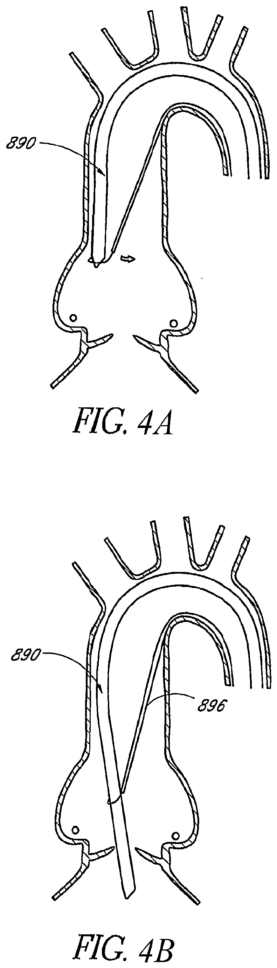

To manipulate the delivery catheter 890 in the lumen of the cardiovascular system, any of a variety of mechanisms or devices may be used. For example, the delivery catheter and/or retaining sheath may comprise a known steering wire that may be actuated by the user at the proximal catheter end to cause bending of the distal catheter tip. In another embodiment of the invention, as exemplified in Figures 4A and 4B, a separate snare 892 may be used to either snare the distal end of the delivery catheter 890 and/or catheter guidewire, which can be pulled to angle or direct the catheter 890 to the desired location or pathway. The snare 892 may be provided in a kit comprising the delivery catheter system and prosthetic valve.

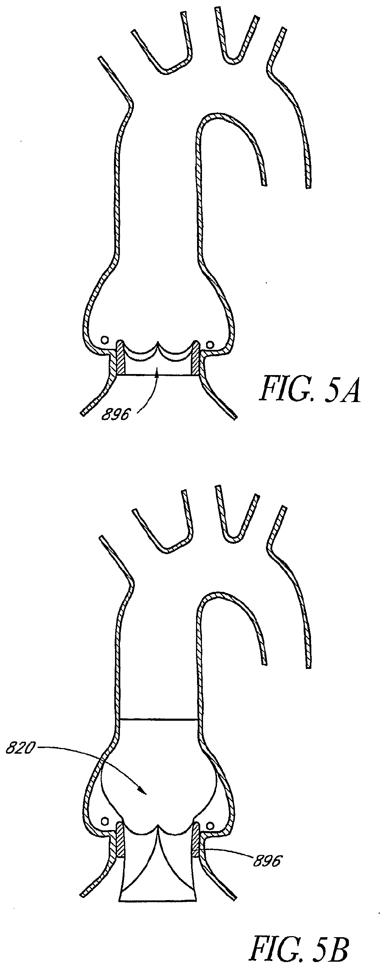

In one embodiment, depicted by example in Figures 5A and 5B, the self-expandable prosthetic valve 894 is implanted about an existing prosthetic valve 896 or prosthetic conduit. The existing prosthetic valve may be a surgically implanted valve 896, as illustrated in Figure 5A, or a minimally invasively inserted valve. A self-expanding prosthetic valve 896 may be better suited for implantation in patients with existing prosthetic valves 896, as illustrated in Figure 5B, because a self-expanding prosthetic valve 894 is adapted to exert sufficient radial force against the existing prosthetic valve in order to seal, anchor and/or provide an adequate lumen diameter at the site of the existing prosthetic valve. In comparison, a balloon-expandable prosthetic valve would likely require a degree of overexpansion such that the final configuration of the prosthetic valve, after recoil following deflation of the balloon, is capable of exerting sufficient force and/or having a final predetermined diameter. However, a pre-existing prosthetic valve will prevent or limit the necessary overexpansion needed to implant a balloon expandable prosthesis at the site of an existing prosthesis because the existing prosthesis lacks the compliance of even sclerotic tissue.

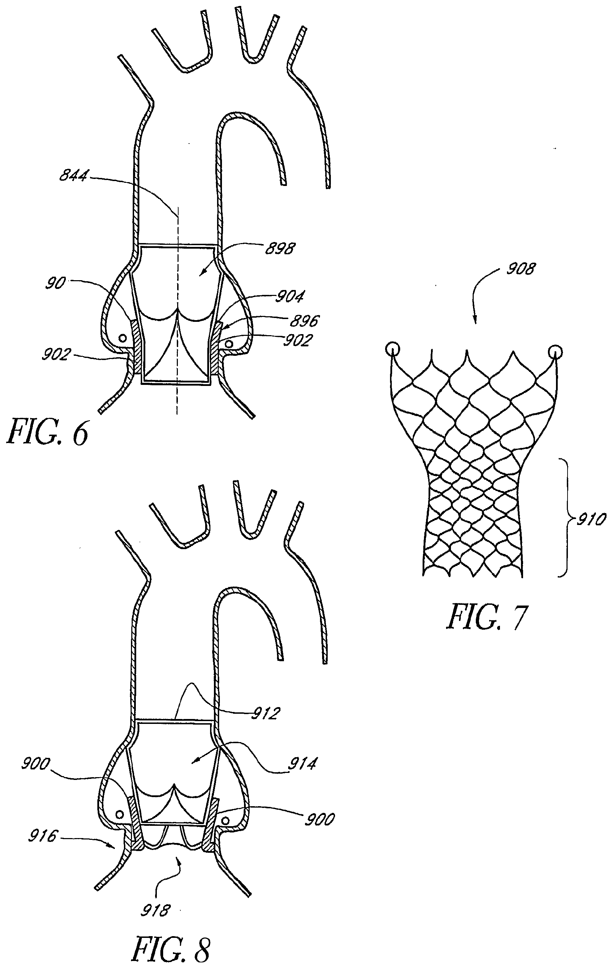

In a further embodiment of the invention, depicted in Figure 6 , an expandable prosthetic valve 898 may be configured for implantation in an existing prosthetic valve 896 or prosthetic conduit such that in addition to pushing aside the valve leaflets of the existing prosthetic valve 896, one or more of the commissure posts 902 of the existing prosthetic valve 896 are deformed or deflected away in order to increase the cross-sectional area of the bloodflow through the expandable prosthetic valve 898. In some embodiments, a balloon catheter or other expansion structure is first applied to one or more of the commissure posts 902 prior to implantation of the expandable prosthetic valve 898 in order to plastically deform the commissure posts 902 and/or to increase the compliance of the commissure posts 902 for expansion by the expandable prosthetic valve 898. In some embodiments, the expandable prosthetic valve 898 is configured to expand with sufficient force to deflect or deform one or more commissure posts 902 without prior application of a balloon catheter. The expandable prosthetic valve 898 may or may not require rotational or angular alignment with the existing prosthetic valve 896 to enhance outward deflection of the commissure posts 902. Angular alignment may be performed by radiography, angiography, intravascular ultrasound or other visualization methods.

Typically the commissure posts 902 are outwardly deflected in a generally radial direction. Not all of the commissure posts 902 need to be deflected or deflected to the same degree or direction. In some embodiments, the ends 904 of one or more commissures posts 902 may be deflected by about 1 mm or more, by about 1.5 mm or more, or preferably by about 2 mm or more. The deflection of the commissure posts may also be measure the the degree of deflection. In some embodiments, the commissure posts 902 may be deflected by about 3 degrees or more, about 5 degrees or more, about 7 degrees or more, about 10 degrees or more, or about 20 degrees or more. In embodiments where the commissure posts 902 of the existing prosthetic valve 896 are oriented in a radially inward direction at rest with respect to the longitudinal axis 844 of the expandable prosthetic valve 898, one or more commissures posts 902 may be deflected to a generally parallel direction or a radially outward direction with respect to the longitudinal axis 844.

Although the shape of the expandable prosthetic valve used in patients where the commissure posts are been deformed or deflected may be similar in shape to the non-cylindrical prosthetic valves described above, in some embodiments the expandable prosthetic valves 908 may have a tapered section 910 configured to wedge against the valve leaflets and/or commissure posts 902 of the existing prosthetic valve 896 and deflect them outwardly.

As illustrated in Figure 8 , the valve frame 912 of expandable prosthetic valve 914 may or may not be configured or dimensioned to anchor or contact the annulus region 916 of the existing native valve 918 when implanted, as the contact against the valve leaflets 900 may be sufficient to anchor the expandable prosthetic valve 914 in place and/or to seal the expandable prosthetic valve 914 against leakage. Likewise, some embodiments of the invention not configured for implantation in an existing prosthetic valve 896 may be similarly configured to anchor/seal at the valve leaflets of the native valve rather that at the annular region of the existing prosthetic valve. It is popularly believed that anchoring against the annulus of the native valve or prosthetic valve is necessary for anchoring of a non-surgically attached prosthetic valve due to the rigidity of the annulus or annular region, but angiographic studies performed with embodiments of the invention suggest that anchoring and or sealing of the expandable prosthetic valve 908 may primarily occur at the valve leaflets. If anchoring at the valve annulus is unnecessary or secondary, a shorter valve frame may be used with minimally invasive or percutaneously inserted prosthetic valves, which may improve the manueverability of the prosthetic valve 908 when loaded on a delivery catheter, thereby facilitating implantation of such devices and reducing the time required to perform the implantation procedure.

## Claims

1. A non-cylindrical prosthetic valve assembly (820) comprising:
a valve and
a prosthesis frame (822) having a first and second end and having a reduced and expanded configuration, the frame (822) comprising:
a first zone being an outflow zone (854) proximal the first end, the first zone (854) including a first plurality of structural members forming a first plurality of cells;
a second zone being an inflow zone (852) proximal the second end, the second zone (852) including a second plurality of structural members forming a second plurality of cells; and
a third zone being a valve support zone (856) between the first and second zones (854, 852), the third zone (856) including a third plurality of structural members forming a third plurality of cells;
wherein the zones (854, 852, 856) are positioned axially with respect to each other, the fully expanded diameter of the first zone is different than that of the second zone and the valve is engaged to the prosthesis frame,
**characterized in that**
the frame consists of adjacent rows of cells from the first to the second end and wherein the cells are largest at the first zone (854), smaller in the second zone (852) and smallest in the third zone (856) and

2. The valve assembly of Claim 1, wherein the fully expanded diameter of the third zone is less than those of the first and second zones.

3. The valve assembly of Claim 2, wherein the third zone comprises a generally concave portion.

4. The valve assembly of Claim 1, wherein the prosthesis frame is self-expanding.

5. The valve assembly of Claim 1, wherein no substantial continuous portion of the prosthesis frame is of constant diameter.

6. The valve assembly of Claim 1, wherein the second end has a diameter less than the greatest fully expanded diameter of the second zone.

7. The valve assembly of Claim 1, wherein the first, second, and third plurality of cells are configured to be expandable.

8. The valve assembly of Claim 1, wherein a portion of the first, second, and third plurality of cells are heterogeneous in structural member configuration.

9. The valve assembly of Claim 1, wherein at least some of the structural members of the first, second, and third zones have varied cross-sectional configurations along their length.

10. The valve assembly of any one of the preceding claims, wherein the valve is a tri-cuspid tissue valve.

## Patentansprüche

1. Nichtzylindrische Klappenprothesenanordnung (820), umfassend:
eine Klappe und
einen Prothesenrahmen (822), der ein erstes und ein zweites Ende aufweist und eine reduzierte und eine ausgedehnte Konfiguration aufweist, der Rahmen (822) umfassend:
eine erste Zone, die eine Ausströmzone (854) proximal des ersten Endes ist, die erste Zone (854) einschließlich einer ersten Vielzahl von Strukturelementen, die eine erste Vielzahl von Zellen bilden;
eine zweite Zone, die eine Einströmzone (852) proximal des zweiten Endes ist, die zweite Zone (852) einschließlich einer zweiten Vielzahl von Strukturelementen, die eine zweite Vielzahl von Zellen bilden; und
eine dritte Zone, die eine Klappenstützzone (856) zwischen der ersten und der zweiten Zone (854, 852) ist, die dritte Zone (856) einschließlich einer dritten Vielzahl von Strukturelementen, die eine dritte Vielzahl von Zellen bilden;
wobei die Zonen (854, 852, 856) hinsichtlich zueinander axial positioniert sind, der vollständig ausgedehnte Durchmesser der ersten Zone sich von dem der zweiten Zone unterscheidet und die Klappe mit dem Prothesenrahmen in Eingriff steht,
**dadurch gekennzeichnet, dass**
der Rahmen aus angrenzenden Reihen von Zellen von dem ersten zu dem zweiten Ende besteht, und wobei die Zellen an der ersten Zone (854) am größten, in der zweiten Zone (852) kleiner und in der dritten Zone (856) am kleinsten sind.

2. Klappenanordnung nach Anspruch 1, wobei der vollständig ausgedehnte Durchmesser der dritten Zone geringer als der der ersten und der zweiten Zone ist.

3. Klappenanordnung nach Anspruch 2, wobei die dritte Zone einen im Allgemeinen konkaven Abschnitt umfasst.

4. Klappenanordnung nach Anspruch 1, wobei der Prothesenrahmen selbstausdehnend ist.

5. Klappenanordnung nach Anspruch 1, wobei kein wesentlicher durchgehender Abschnitt des Prothesenrahmens von einem konstanten Durchmesser ist.

6. Klappenanordnung nach Anspruch 1, wobei das zweite Ende einen Durchmesser, der geringer als der größte, vollständig ausgedehnte Durchmesser der zweiten Zone ist, aufweist.

7. Klappenanordnung nach Anspruch 1, wobei die erste, die zweite und die dritte Vielzahl von Zellen konfiguriert sind, um ausdehnbar zu sein.

8. Klappenanordnung nach Anspruch 1, wobei ein Abschnitt der ersten, der zweiten und der dritten Vielzahl von Zellen in Strukturelementkonfiguration heterogen ist.

9. Klappenanordnung nach Anspruch 1, wobei mindestens einige der Strukturelemente der ersten, der zweiten und der dritten Zone entlang ihrer Länge verschiedene Querschnittskonfigurationen aufweisen.

10. Klappenanordnung nach einem der vorstehenden Ansprüche, wobei die Klappe eine Trikuspidalgewebeklappe ist.

## Revendications

1. Ensemble valve prothétique non cylindrique (820) comprenant :
une vanne et
un cadre de prothèse (822) ayant une première et une seconde extrémité et ayant une configuration réduite et déployée, le cadre (822) comprenant :
une première zone étant une zone de sortie (854) proximale de la première extrémité, la première zone (854) comportant une première pluralité d'éléments structurels formant une première pluralité de cellules ;
une deuxième zone étant une zone d'entrée (852) proximale de la seconde extrémité, la deuxième zone (852) comportant une deuxième pluralité d'éléments structurels formant une deuxième pluralité de cellules ; et
une troisième zone étant une zone de support de valve (856) entre les première et deuxième zones (854, 852), la troisième zone (856) comportant une troisième pluralité d'éléments structurels formant une troisième pluralité de cellules ;
dans lequel les zones (854, 852, 856) sont positionnées axialement les unes par rapport aux autres, le diamètre complètement déployé de la première zone est différent de celui de la deuxième zone et la valve est en prise avec l'armature de la prothèse,
**caractérisé en ce que**
le cadre est constitué de rangées adjacentes de cellules de la première à la seconde extrémité et dans lequel les cellules sont les plus grandes au niveau de la première zone (854), plus petites dans la deuxième zone (852) et les plus petites dans la troisième zone (856).

2. Ensemble valve selon la revendication 1, dans lequel le diamètre entièrement déployé de la troisième zone est inférieur à ceux des première et deuxième zones.

3. Ensemble valve selon la revendication 2, dans lequel la troisième zone comprend une partie généralement concave.

4. Ensemble valve selon la revendication 1, dans lequel le cadre de prothèse est auto-déployable.

5. Ensemble valve selon la revendication 1, dans lequel aucune partie continue substantielle du cadre de prothèse ne présente de diamètre constant.

6. Ensemble valve selon la revendication 1, dans lequel la seconde extrémité a un diamètre inférieur au plus grand diamètre complètement déployé de la deuxième zone.

7. Ensemble valve selon la revendication 1, dans lequel les première, deuxième et troisième pluralités de cellules sont conçues pour être déployables.

8. Ensemble valve selon la revendication 1, dans lequel une partie de la première, deuxième et troisième pluralité de cellules est hétérogène en configuration d'élément structurel.

9. Ensemble valve selon la revendication 1, dans lequel au moins certains des éléments structurels des première, deuxième et troisième zones présentent des configurations en coupe transversale variées sur leur longueur.

10. Ensemble valve selon l'une quelconque des revendications précédentes, dans lequel la valve est une valve tricuspide en tissu.
